# EUROPÄISCHE PATENTSCHRIFT

(11) **EP 2 079 849 B1**
(45) Veröffentlichungstag und Bekanntmachung des Hinweises auf die Patenterteilung: **20.11.2013**
(21) Anmeldenummer: 07819390.1
(22) Anmeldetag: 27.10.2007
(51) Int. Cl.: C12Q 1/56, G01N 33/86

(54) **VERFAHREN UND VORRICHTUNGEN ZUR ELEKTROCHEMISCHEN BESTIMMUNG VON FAKTOR XA-INHIBITOREN IN BLUTPROBEN**
METHODS AND APPARATUSES FOR ELECTROCHEMICAL DETERMINATION OF FACTOR XA INHIBITORS IN BLOOD SAMPLES
PROCÉDÉ ET DISPOSITIFS POUR LA DÉTERMINATION ÉLECTROCHIMIQUE D'INHIBITEURS DU FACTEUR XA DANS DES ÉCHANTILLONS DE SANG

(30) Priorität: 31.10.2006 EP 06123234
(43) Veröffentlichungstag der Anmeldung: 22.07.2009
(73) Patentinhaber: Roche Diagnostics GmbH, 68305 Mannheim (DE); F.Hoffmann-La Roche AG, 4070 Basel (CH)
(72) Erfinder: HORN, Carina, 68647 Biblis (DE); DOERGE, Liesel, 69250 Schönau (DE); GALM, Margarete, 82515 Wolfratshausen (DE)
(74) Vertreter: Immendörfer-Rühle, Ulrike
(86) Internationale Anmeldenummer: PCT/EP2007/009347
(87) Internationale Veröffentlichungsnummer: WO 2008/052718

(56) Entgegenhaltungen:
- WO-A-00/77246
- WO-A-01/63271
- WO-A-92/07954
- WO-A-93/10262
- WO-A-2005/031303
- DE-T2- 60 024 669
- US-A1- 2006 166 284

## Beschreibung

### Gebiet der Erfindung

Die Erfindung betrifft Verfahren zur elektrochemischen Bestimmung von Faktor Xa-Inhibitoren, insbesondere von Heparinen und Heparinderivaten sowie direkten Faktor Xa-Inhibitoren, in Blutproben.

Weiterhin betrifft die Erfindung Testelemente auf trockenchemischer Basis und Testelement-Analysesysteme zur elektrochemischen Bestimmung von Faktor Xa-Inhibitoren, insbesondere von Heparinen und Heparinderivaten sowie direkten Faktor Xa-Inhibitoren, in Blutproben.

### Stand der Technik:

Zur Prophylaxe und Therapie von hämostatischen Störungen (Störungen des Gerinnungssystems) werden in der klinischen Praxis oft Antikoagulantien eingesetzt, wozu insbesondere auch Heparine zählen. Heparine und Heparinderivate werden insbesondere zur Therapie und Prophylaxe von thromboembolischen Erkrankungen verwendet. Sie sind sehr effektiv bei der Prophylaxe und Behandlung von Beinvenenthrombosen, Lungenembolien und zur Behandlung von instabiler Angina pectoris und akutem Myokardinfarkt. Vielfachen Einsatz finden sie auch während Operationen, insbesondere bei kardiologischen Eingriffen (Bypass) und bei Bluttransfusionen.

Die Wirkung der Heparine beruht hauptsächlich auf ihrer Interaktion mit Antithrombin III (ATIII), wodurch sie eine Konformationsänderung des ATIII bewirken. Hierdurch wird die Inaktivierung bestimmter Gerinnungsenzyme (Thrombin (FIIa), Faktor Xa (FXa) und Faktor IXa) beschleunigt und somit die Gerinnung verzögert. Heparine können somit in die Klasse der Faktor Xa-Inhibitoren eingeordnet werden. Weitere Faktor Xa-Inhibitoren sind beispielsweise bestimmte Oligosaccharide wie das Pentasaccharid Fondaparinux oder sich noch in der klinischen Entwicklung befindliche niedermolekulare direkte Faktor Xa-Inhibitoren, welche verschiedenen Substanzklassen zugeordnet werden können. Neben den seit langer Zeit eingesetzten unfraktionierten Heparinen (UFH=unfractioned heparin) werden seit Ende der 1980er Jahre auch verschiedene fraktionierte Heparine oder Low Mölecular Weight Heparine (=LMWH) eingesetzt. Fraktionierte Heparine ersetzen mittlerweile für viele Indikationen die UFH und werden durch chemische oder enzymatische Depolymerisation aus unfraktionierten Heparinen hergestellt, wobei Fragmente entstehen, die nur ca. 1/3 der Größe des Standard-Heparins haben. Hierdurch wird u.a. die Wirkung dieser LMWHs auf Thrombin abgeschwächt, während die Inaktivierung von Faktor Xa bevorzugt ist. Fraktionierte Heparine besitzen aufgrund ihrer vorteilhafteren Pharmakokinetik weitere Vorteile gegenüber den konventionellen unfraktionierten Heparinen. Ein Übersicht über diese Substanzklassen hinsichtlich ihrer klinischen Wirkung und Bedeutung ist in "Heparin and Low-Molecular-Weight Heparin (Mechanisms of Action, Pharmakokinetics, Dosing, Monitoring, Efficacy, and Safety)", Hirsh et al.; Chest 2001; 119:64S-94S zu finden.

Für Patienten, denen unfraktioniertes Heparin verabreicht wird, ist ein Monitoring aufgrund der individuellen Variabilität bei der Bioverfügbarkeit, der Proteinbindung und einer kurzen Halbwertszeit von 30-150 min vorgeschrieben, um eine eventuelle Überdosierung mit erhöhter Blutungsneigung oder Unterdosierung mit erhöhtem Thromboserisiko zu vermeiden. Die Überwachung der Therapie mit UFH erfolgt in der klinischen Routine am häufigsten mit der aktivierten partiellen Thromboplastinzeit (aPTT), aber auch mittels der Thrombin Clotting Time (TCT) oder der aktivierten Clotting Time (ACT). Diese Assays sind so genannte globale Assays, da sie unspezifisch die Thrombin-induzierte Bildung eines Fibrin-Clots reflektieren. Der aPTT-Test, welcher v.a. die Aktivität der Faktoren des intrinsischen Systems bestimmt, ist überwiegend sensitiv für die inhibierenden Effekte des Heparins auf Thrombin. Der aPTT-Test ist empfindlich für den Heparinbereich von 0,1-0,7 U/ml, wobei jedoch sowohl der Normbereich der aPTT als auch deren therapeutischer Bereich sehr stark vom Reagenz und dem verwendeten Analysegerät abhängig ist. Ein weiterer limitierender Faktor ist die Probenstabilität, die besonders nach zu langer Standzeit der Blutprobe zu oftmals verfälschten Ergebnissen führt. Weitere Nachteile solcher globaler Gerinnungsassays sind u.a. die oft komplexe Versuchsdurchführung, welche zur Erzielung reproduzierbarer Ergebnisse speziell geschultes Personal erfordert und der relative hohe Reagenzienverbrauch dieser Tests.

Aufgrund der besseren Pharmakokinetik ist ein Monitoring bei Gabe niedermolekularer Heparine beim Normalpatienten nicht zwingend. Eine Überprüfung der Therapie wird jedoch zu Beginn einer Behandlung empfohlen und ist insbesondere bei niereninsuffizienten Patienten aufgrund ihrer veränderten renalen Ausscheidung erforderlich sowie empfohlen bei Patienten mit extremem Körpergewicht, Neugeborenen, Kindern und Schwangeren oder bei mehrwöchiger Anwendung bzw. nach frischen Traumen oder Operationen.

In der klinischen Routine findet für das Monitoring von LMWH vor allem die aPTT Anwendung, obwohl dieser Test nur unzureichend sensitiv für dieses Antikoagulanz ist und zudem noch in sehr starkem Maße vom benutzten Nachweisreagenz abhängt.

Ein für ein Monitoring der Wirkung niedermolekularer Heparine geeigneter Nachweis-Test ist ein FXa-Test.

Bisherige FXa-Tests werden zumeist als chromogene Tests oder als Clotting Tests durchgeführt, wobei die chromogenen Tests Faktor-Xa-Aktivität und der Clotting Test Gerinnung messen. Beide Testprinzipien folgen demselben Testablauf:
1. FXa + [Heparin/Antithrombin III] → [FXa/Heparin/ATIII]-Komplex + restlicher FXa
2.
   a) restlicher FXa spaltet von FXa-spezifischem Substrat einen chromogenen Rest ab (chromogener Test)
   b) restlicher FXa spaltet Prothrombin zu Thrombin (Gerinnungstest über thrombininduzierte Fibrinvernetzung)

Bei Zugabe der Probe verbindet sich in definierter Menge im Testreagenz vorhandener Faktor Xa mit dem in der Probe enthaltenen Heparin und Antithrombin III und bildet mit diesen einen inaktivierten Komplex. Der restliche Faktor Xa spaltet entweder das chromogene Substrat oder bildet mit den übrigen in der Probe enthaltenen Gerinnungsfaktoren Thrombin, das Fibrinogen zu Fibrin spaltet (Clotbildung). Je nach Aktivität des Faktors Xa wird mehr oder weniger Substrat gespalten. Die Aktivität des Faktor Xa ist wiederum abhängig von der Menge des in der Probe enthaltenen Heparins. Während die chromogenen Tests spezifisch für die FXa-Aktivität in der Probe sind, messen Gerinnungstests nicht ausschließlich die FXa-Aktivität, sind jedoch oft dennoch sensitiver für LMWH als die aPTT.

Kommerziell sind mehrere chromogene Tests, aber nur wenige Gerinnungstests (Heptest der Fa. Sigma, ENOX-Test der Fa. Pharmanetics) verfügbar. Die verschiedenen Tests korrelieren untereinander und zur aPTT nur mäßig, da sie unterschiedliche Endpunkte haben. Die chromogenen Tests liefern Aktivitäten, keine Gerinnungszeiten. Die Korrelation zwischen Gerinnungszeit und Faktor Xa-Aktivität ist jedoch nur mäßig. Weiterhin erfordern diese chromogenen Tests eine Separation des Plasmas und können somit nicht direkt in Vollblutproben eingesetzt werden. Durch die aufwändigen Probenvorbereitungs- und Durchführungsschritte und die benötigten Vorrichtungen erfordern diese Bestimmungsmethoden einen hohen zeitlichen, personellen und apparativen Aufwand. Der Heptest liefert als Ergebnis zwar eine Gerinnungszeit, diese kann jedoch aufgrund der unterschiedlichen Heparinsensitivität des Patienten bei gleicher Heparinkonzentration sehr unterschiedlich ausfallen. Weiterhin ist die Erstellung einer Heparin-Eichkurve erforderlich, aus der dann das Testresultat abgelesen wird. Als trockenchemischer Test und somit auch für Point of Care-Geräte geeignet ist bislang nur ein einziger Faktor Xa-Test verfügbar (ENOX-Test der Fa. Pharmanetics), der mit einer Testkarte auf dem Rapid Point Analyzer der Fa. Bayer durchgeführt wird. Dieser Test wurde speziell für den Einsatz von Enoxaparin bei der Perkutanen Transluminalen Angioplastie entwickelt und unterscheidet lediglich zwischen Enoxaparin-Konzentrationen > 1 U/ml und <1 U/ml und ist somit für das Routinemonitoring niedermolekularer Heparine nicht geeignet, da insbesondere andere fraktionierte Heparine und unfraktioniertes Heparin den Test stören.

WO 92/07954 beschreibt Verfahren und Vorrichtungen für einen nasschemischen Test zur Konzentrationsbestimmung von Antikoagulantien in verdünnten Flüssigkeitsproben, in deren der verdünnten Flüssigkeitsprobe nacheinander ein Gerinnungsfaktor (z.B. Faktor X), ein Aktivator-Reagenz und ein Nachweis-Reagenz zugesetzt, welches ein Thrombin-Substrat ist.

WO 03/050298 beschreibt das Prinzip dieses trockenchemischen FXa-Tests wie folgt: Die zu untersuchende Probe, vorzugsweise citriertes Vollblut, wird mit einem trockenchemischen Reagenz versetzt, welches zumindest einen Faktor Xa-Aktivator, vorzugsweise Russels Viper Venom, sowie homogen verteilte magnetische Partikel enthält. Durch den im Reagenz enthaltenen Faktor Xa-Aktivator wird der in der Probe enthaltene Faktor X in Faktor Xa umgesetzt, welcher seinerseits über die Prothrombin-Thrombin-Umwandlung die Fibrinogenumsetzung zu Fibrin und damit die Ausbildung des Gerinnungs-Clots bewirkt. Der Nachweis dieses Clots erfolgt mittels optischer Methoden durch Beobachtung der Beweglichkeit der magnetischen Partikel im Reaktionsansatz, welche durch ein externes oszillierendes Magnetfeld hervorgerufen wird. Dieses Testprinzip beruht somit auf der Bildung eines Fibrin-Clots, welcher sich erst im Laufe der Nachweisreaktion in einer mehrstufigen, durch Faktor Xa ausgelösten Reaktionskaskade bildet, an welcher neben Faktor Xa weitere Enzyme und Cofaktoren beteiligt sind. So erfordert beispielsweise die Polymerisation und Quervernetzung des Fibrins die Anwesenheit von Calcium-Ionen und aktiviertem Faktor XIIIa, welcher wiederum durch eine Thrombin-abhängige Aktivierung aus inaktivem Faktor XIII gebildet wird. Die Bestimmung von Faktor Xa mittels der Bestimmung des Fibrin-Clots ist somit noch von weiteren essentiellen Faktoren und eventuellen Störeinflüssen abhängig. Neben dieser indirekten Bestimmungsmethode erfordert das in WO 03/050298 dargestellte Nachweisverfahren ein komplexes Nachweis- und Auswertesystem zur Faktor Xa-Bestimmung. So muss einerseits der Testträger, auf welchem die Gerinnungsreaktion stattfindet, spezielle Vorrichtungen aufweisen, welche eine gute und homogene Durchmischung der Reagenzien und Magnetpartikel mit der Probe gewährleisten und andererseits muß das Auswertesystem zur Bestimmung der Faktor Xa-Aktivität Vorrichtungen zur Erzeugung eines oszillierenden Magnetfelds, beispielsweise mittels eines beweglichen Permanentmagneten, und optische Systeme zur Beleuchtung und photometrischen Detektion der Bewegung der Magnetpartikel aufweisen.

WO 01/63271 beschreibt allgemein elektrochemische Sensoren auf trockenchemischer Basis zur Bestimmung der Blutgerinnung oder einzelner Gerinnungsfaktoren, welche auf einem inerten Träger mindestens 2 Elektroden aufweisen, sowie ein trockenes Reagens, welches ein Proteasesubstrat enthält, das aus einem Peptidrest besteht, der von einer Protease des Blutgerinnungssystems abgespalten werden kann, und über das Carboxylende amidisch an substituierte Amine, insbesondere an einen Phenylendiaminrest, gebunden ist. Diese substituierten Amine wirken nach der Protease-induzierten Abspaltung als Elektronenüberträger 2. Art und können zur elektrochemischen Bestimmung der Proteaseaktivität herangezogen werden. WO 01/63271 beschreibt neben den so genannten Globaltests wie aPTT, PT oder ACT, bei welchen die Gerinnungszeit über die Aktivität der Protease Thrombin ermittelt wird, auch Tests, mit welchen einzelne Gerinnungsfaktoren oder auch deren Inhibitoren bestimmt werden können. Hier lehrt WO 01/63271 den Einsatz von speziell auf den zu bestimmenden Gerinnungsfaktor zugeschnittenen Substraten, deren Peptidteil speziell an die zu bestimmende Protease angepasst ist, so dass es von dieser Protease spezifisch gespalten werden kann und das substituierte Amin als elektrochemisch detektierbares Teilchen spezifisch die Aktivität dieser Protease widerspiegelt. Übertragen auf einen Faktor Xa-Test würde dies bedeuten, ein Proteasesubstrat einzusetzen, welches aus einem Peptidrest besteht, der von Faktor Xa abgespalten werden kann, und über das Carboxylende amidisch an substituierte Amine, insbesondere an einen Phenylendiaminrest gebunden ist. Hierin ähnelt das Test-Prinzip den chromogenen Faktor Xa-Tests, bei welchen ebenfalls ein enzymatisches Abspaltungs-Produkt eines Faktor Xa-spezifischen Substrats zur Faktor Xa-Bestimmung eingesetzt wird.

### Aufgabe der Erfindung:

Aufgabe der vorliegenden Erfindung ist es, Verfahren und Vorrichtungen zur Bestimmung von Faktor Xa-Inhibitoren in Blutproben bereitzustellen, welche die Nachteile des Standes der Technik vermeiden.

Aufgabe der vorliegenden Erfindung ist es insbesondere, Verfahren zur Bestimmung von Faktor Xa-Inhibitoren in Blutproben bereitzustellen, welche einfach und mit geringem zeitlichen, apparativen oder personellem Aufwand auch von nicht speziell geschulten Personen durchgeführt werden können und in kurzer Zeit zu verlässlichen Ergebnissen führen.

Aufgabe der vorliegenden Erfindung ist es insbesondere, einfach durchzuführende Verfahren zur Bestimmung von Faktor Xa-Inhibitoren in Blutproben bereitzustellen, welche mit einer möglichst kleinen Zahl von Verfahrensschritten und benötigten Reagenzien und/oder Apparaten auskommt, so dass eine rasche dezentrale Analytik, beispielsweise direkt auf Intensiv- oder Krankenstationen, möglich wird.

Aufgabe der vorliegenden Erfindung ist es insbesondere, Verfahren und Vorrichtungen zur Bestimmung von Faktor Xa-Inhibitoren in Blutproben bereitzustellen, welche eine Bestimmung auch direkt im Vollblut ermöglichen und somit keine aufwändigen Probenvorbereitungsschritte benötigen.

Aufgabe der vorliegenden Erfindung ist es insbesondere, Verfahren und Vorrichtungen zur Bestimmung von Faktor Xa-Inhibitoren in Blutproben bereitzustellen, welchen den Anforderungen an Lagerbarkeit und Stabilität der Reagenzien genügen und eine möglichst genaue und spezifische Bestimmung ermöglichen.

Aufgabe der vorliegenden Erfindung ist es insbesondere, Verfahren und Vorrichtungen zur Bestimmung von Heparinen, insbesondere fraktionierten Heparinen oder Low Molecular Weight Heparinen, sowie direkten Faktor Xa-Inhibitoren in Blutproben bereitzustellen.

### Erfindungsgemäße Lösung:

Die vorliegende Erfindung stellt zur Lösung dieser Aufgaben Verfahren, elektrochemische Testelemente und Testelement-Analysesysteme zur Bestimmung von Faktor Xa-Inhibitoren in Blutproben bereit, wie sie in den unabhängigen Patentansprüchen genannt sind. Bevorzugte Ausführungsformen sind in den abhängigen Ansprüchen angegeben.

Insbesondere beschreibt die vorliegende Erfindung zur Lösung dieser Aufgaben Verfahren zur Bestimmung von Faktor Xa-Inhibitoren in Blutproben, welche dadurch gekennzeichnet sind, dass in einem ersten Schritt eine Probe des zu untersuchenden Blutes mit einem Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, enthält, und mit einer bekannten Menge von Faktor Xa in Kontakt gebracht wird, anschließend in einem zweiten Schritt die Menge oder Aktivität der elektrogenen Substanz, welche durch die Faktor Xa-vermittelte Thrombinaktivierung vom Thrombinsubstrat abgespalten wird, und/oder deren zeitlicher Verlauf als Messsignal mit elektrochemischen Methoden bestimmt wird, und schließlich in einem dritten Schritt anhand dieses Messsignals die Menge des Faktor Xa-Inhibitors in der Probe des zu untersuchenden Blutes oder eine damit korrelierende Messgröße, insbesondere eine damit korrelierende Gerinnungszeit, ermittelt wird.

Erfindungsgemäß wird die bekannte Menge von Faktor Xa im ersten Verfahrensschritt nicht direkt als Faktor Xa-Reagenz zugesetzt, sondern erfolgt durch Zugabe eines Reagenz enthaltend eine bekannte Menge von Faktor X (Faktor X- Reagenz) und eines Aktivator-Reagenz, welche die Umwandlung von Faktor X in Faktor Xa hervorruft, zur Probe in einem gemeinsamen Schritt.

Das erfindungsgemäße Verfahren wird anhand eines exemplarischen Beispiels näher erläutert:

Das Testprinzip beruht auf der Bestimmung der Enzymaktivität des durch die Faktor Xa-induzierte Gerinnungsreaktion gebildeten Thrombins mittels einer elektrochemischen Messung, wobei zumindest ein Spaltprodukt elektrochemisch an den Elektroden bestimmt wird. Als Thrombin-spezifisches Substrat kann insbesondere ein Tripeptid-Substrat wie beispielsweise reduziertes Chromozym TH (Tosyl-glycyl-prolyl-arginine-4-nitranilide acetate) eingesetzt werden, welches über das Carboxylende des Tripeptidteils amidisch eine elektrogene Substanz gebunden hat. Solche elektrogenen Substanzen sind beispielsweise in WO 01/63271 beschrieben und können insbesondere substituierte Aniline, insbesondere Nitroanilinderivate oder Phenylendiamine, sein. Die Nachweisreaktion läuft folgendermaßen ab:

Das Enzym Thrombin spaltet vom Substrat eine elektrogene Substanz, im vorliegenden Beispiel Phenylendiamin, ab, welches an den Elektroden zu Phenylendiimin oxidiert wird. In einer bevorzugten Ausführungsform findet an den Elektroden gleichzeitig mit der Oxidation dieses Spaltproduktes eine Reduktion einer Hilfssubstanz, beispielsweise von Resazurin zu Resorufin, statt, wodurch eine Stromstärke gemessen werden kann, welche mit der Spaltproduktmenge korreliert. So wird Resazurin in der vorliegenden elektrochemischen Reaktion als Hilfssubstanz an der Kathode reduziert, während an der Anode Phenylendiamin oxidiert wird. Für die elektrochemische Messung der Thrombinaktivität wird vorzugsweise ein Zwei-Elektroden-System eingesetzt, wobei das Potential an der Arbeitselektrode einerseits so hoch gewählt wird, dass das von Thrombin freigesetzte Phenylendiamin oxidiert wird und andererseits so niedrig gewählt wird, dass keine Restgruppe des Tripeptids reduziert wird. Die Potentialdifferenz zwischen der Kathode (Arbeitselektrode) und der Anode (Gegenelektrode) wird durch einen Potentiostaten geregelt. Als geeignete Potentialdifferenz kann im vorliegenden Beispiel beispielsweise eine Spannungsdifferenz von ca. 550 mV gewählt werden, um nur die gewünschten Reaktionen an den Elektroden zu ermöglichen.

In Bezug auf das erfindungsgemäße Verfahren wird dieses elektrochemische Testprinzip in einem Faktor Xa-Inhibitor-Test wie folgt angewendet:

Im Reaktionsansatz befindet sich neben den Nachweisreagenz eine bekannte Menge an Faktor Xa, welche durch Zugabe einer bekannten Menge von Faktor X-Reagenz und eines Aktivator-Reagenz, welche die Umwandlung von Faktor X in Faktor Xa hervorruft, entstanden ist. Die zu untersuchende Blutprobe enthält den zu bestimmenden FXa-Inhibitor, welcher vorzugsweise ein Heparin ist. Dieses bildet mit dem im Reaktionsansatz befindlichen und in bekannter Menge im Überschuß vorliegenden Faktor Xa und Antithrombin III, welches in der Blutprobe vorliegt, einen stöchiometrischen Komplex. Hierbei bleibt eine FXa-Restmenge übrig, deren Konzentration von der Konzentration des Faktor Xa-Inhibitors in der Probe abhängig ist. Diese kann nun mit dem in der Blutprobe vorliegenden Prothrombin (und dem auch dort vorliegenden Faktor Va) zum Prothrombinkomplex und anschließend zum Thrombin reagieren, welches wie oben beschrieben über die thrombinbedingte enzymatische Abspaltung einer elektrogenen Substanz und deren elektrochemischen Nachweis bestimmt werden kann.

Je mehr Faktor Xa-Inhibitor in der Probe vorliegt, desto weniger Rest-FXa bleibt nach der Komplexbildung mit ATIII übrig und desto weniger Thrombin wird gebildet. Das vorliegende Messprinzip detektiert keine Gerinnungszeit im strikten Sinne, d.h. es handelt sich nicht um einen Clotting Test mit der Bildung von Fibrin, sondern um eine Bestimmung des zeitlichen Verlaufs der Thrombinbildung mit elektrochemischen Methoden.

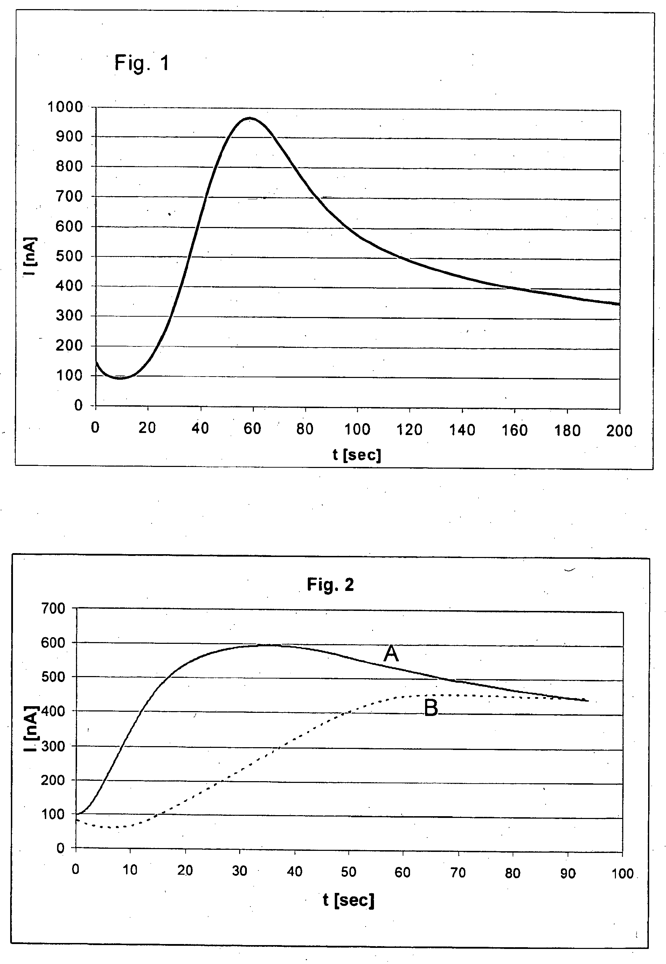

Beispiel 2 und Figur 1 zeigen exemplarisch das Ergebnis einer solchen elektrochemischen Thrombinbestimmung entsprechend den erfindungsgemäßen Verfahren.

Die erfindungsgemäße Verwendung von aktiviertem Faktor Xa mit einem Thrombin-Nachweisreagenz besitzt im Vergleich zu den chromogenen Faktor Xa-Tests und der in WO 01/63271 beschriebenen Verfahren eine höhere Aussagekraft über den physiologischen Zustand des Gerinnungssystems, da hier nicht ein künstliches Faktor Xa-spezifisches Substrat gespalten und darüber die Faktor Xa-Aktivität gemessen wird, sondern wie in der physiologischen Gerinnung zuerst Thrombin gebildet wird. Diese Bildung von Thrombin kann erfindungsgemäß mittels eines Thrombin-spezifischen Substrats elektrochemisch bestimmt und mit einem geeigneten Algorithmus beispielsweise in Gerinnungszeiten umgerechnet werden. Gegenüber Faktor Xa-Inhibitortests, welche wie der Heptest oder der ENOX-Test auf der Bestimmung der FXa-induzierten Bildung eines Fibrin-Clots beruhen, hat die Erfindung den Vorteil, dass die Bestimmung mittels elektrochemischer Methoden wesentlich einfacher und mit weniger personellem und apparativem Aufwand durchgeführt werden kann. Dadurch, dass die Faktor Xa-Aktivität mit den erfindungsgemäßen Verfahren durch die Aktivität des ersten in der physiologischen Gerinnungskaskade natürlicherweise vorkommenden nachgeschalteten Gerinnungsfaktors (Thrombin) bestimmt wird, wird einerseits die physiologische Aktivität des Faktor Xa-Inhibitors näher am natürlichen System bestimmt als mit chromogenen Tests und den in WO 01/63271 beschriebenen Verfahren, welche mit unphysiologischen Faktor Xa-Substraten arbeiten und andererseits wird der Nachweis der FXa-Aktivität an einer früheren Stelle der Gerinnungskaskade als bei den Tests, welche auf der Bestimmung der FXa-induzierten Bildung eines Fibrin-Clots beruhen, durchgeführt, so dass die zwischen der Thrombinbildung und der Fibrin-Clot-Bildung befindlichen, nachgeschalteten Gerinnungsfaktoren keinen Einfluss auf die Bestimmung der Faktor Xa-Aktivität haben. Hierdurch sind somit genauere und spezifischere Aussagen über die Faktor Xa-Aktivität im physiologischen System möglich als mit den bisher bekannten Tests.

Durch die erfindungsgemäße Lösung ist eine Faktor Xa-Inhibitor-Bestimmung insbesondere auch im Vollblut möglich, da anders als bei chromogenen Messmethoden eine Abtrennung störender, insbesondere farbiger, Blutbestandteile bei den genutzten elektrochemischen Methoden nicht notwendig ist. Durch eine auf die zum Nachweis genutzten elektrogenen Substanzen abgestimmte Ansteuerung der Elektroden, beispielsweise durch angepasste Wahl des Elektrodenpotentials, können die zum Nachweis genutzten elektrogenen Substanzen weitgehend unbeeinflusst von anderen in der Probe oder den Reagenzien vorkommenden und potentiell störenden Substanzen nachgewiesen werden.

Zur Bestimmung eines Faktor Xa-Inhibitors müssen erfindungsgemäß der zu untersuchenden Probe zumindest folgende Substanzen zugefügt werden: eine bekannte Menge von Faktor X, ein Aktivator-Reagenz und als Nachweisreagenz ein peptidisches Thrombinsubstrat, aus welchem enzymatisch eine elektrogene Substanz abgespalten werden kann. Die Zugabe der bekannten Menge von Faktor X und des Aktivator-Reagenzerfolgt in einem gemeinsamen Schritt. Viele der bekannten und in Thrombin-Tests eingesetzten Thrombinsubstrate, darunter auch das verbreitet eingesetzte Chromozym TH, weisen jedoch keine absolute und ausschließliche Thrombinspezifität auf, sondern können auch von anderen Enzymen gespalten werden.

Der Gerinnungsfaktor Xa ist eine Serinprotease, die natürlicherweise die Aminosäuresequenz -Ile-Glu-Gly-Arg- des Prothrombins erkennt und durch Spaltung an dieser Sequenz das natürliche Substrat Prothrombin zu Thrombin aktiviert. Neben diesem physiologischen Substrat hat Faktor Xa auch eine Fähigkeit, andere Peptide mit dieser oder auch anderen Spaltsequenzen enzymatisch zu spalten. So können auch peptidische Thrombinsubstrate, insbesondere das verbreitet eingesetzte Chromozym TH, von Faktor Xa, gespalten werden. Durch diese geringe Substratspezifität des Faktors Xa können in den erfindungsgemäßen Verfahren insbesondere dann Probleme auftreten, wenn ein solches auch von Faktor Xa spaltbares Thrombinsubstrat als Nachweisreagenz verwendet wird und dieses vor Zugabe der Probe bereits mit Faktor Xa in Kontakt kommt und von diesem umgesetzt werden kann. Hierdurch kann bereits ein Teil des Thrombinsubstrats umgesetzt werden, so dass dieser nicht mehr für die Thrombin-vermittelte Nachweisreaktion zur Verfügung steht und somit das Messresultat beeinflusst wird. Solche unerwünschten Nebenreaktionen können insbesondere dann auftreten, wenn Faktor Xa und das Thrombinsubstrat über einer längeren Zeitraum in Kontakt sind, wie dies beispielsweise der Fall wäre, wenn man diese beiden Substanzen gleichzeitig in ein flüssiges Nachweis-Reagenz bringen würde.

Bezogen auf das zuvor genannte exemplarische Messsystem würde dies bedeuten, dass im Falle, dass Faktor Xa und das als Thrombinsubstrat eingesetzte reduzierte Chromozym TH vor Beginn der Nachweisreaktion bereits über einen längeren Zeitraum miteinander reagieren konnten, bereits ein Teil des Thrombinsubstrats gespalten wäre und somit bereits eine weitgehend undefinierte Menge der abgespaltenen elektrogenen Substanz im Reagenz vorliegen würde. Solche Fälle können insbesondere dann vorliegen, wenn die beiden Stoffe gemeinsam in einem Flüssigreagenz vorliegen oder im Falle trockenchemischer Tests während derer Herstellung in flüssiger Form in Kontakt kamen. So kann eine gemeinsame Standzeit von 2h bereits ausreichen, um ca. 25% des vorhandenen reduzierten Chromozyms TH alleine durch die Faktor Xa-Wirkung zu spalten. Die Folge eines solchen Faktor Xa-bedingten vorzeitigen Substratabbaus wäre bei den elektrochemischen erfindungsgemäßen Verfahren insbesondere, dass das Messsignal bereits vor Beginn der Thrombin-bedingten Spaltung des reduzierten Chromozyms TH einen Versatz oder Offset zu höheren Stromstärken sowie auch hohe Stromstärken am Minimum aufweist, da in der Probe bereits durch die enzymatische Wirkung des Faktors Xa freigesetztes Phenylendiamin als elektrogene Substanz vorhanden ist.

Erfindungsgemäß wird die Problematik des vorzeitigen Faktor Xa-bedingten Substratabbaus dadurch zumindest teilweise gelöst, dass eine bekannte Menge von Faktor Xa im Reaktionsansatz durch Zugabe einer bekannten Menge von Faktor X-Reagenz und eines Aktivator-Reagenz, welche die Umwandlung des proteolytisch inaktiven Faktors X in den proteolytisch aktiven Faktor Xa hervorruft, zur Probe erreicht wird.

Überraschenderweise hat sich durch dieses erfindungsgemäße Verfahren gezeigt, dass durch diese Reagenzienkombination ein besonders einfacher und stabiler Testelementaufbau möglich ist. Durch die Tatsache, dass im Reagenz vor dem Zusatz bzw. der Aktivierung eines Aktivator-Reagenzes kein aktivierter Faktor Xa, sondern inaktiver Faktor X vorliegt, ist es möglich, das Nachweisreagenz bereits vor Beginn der Bestimmungsreaktion mit Faktor X in Kontakt zu bringen, ohne dass es zu einem vorzeitigen Faktor Xa-bedingten Substratabbau und damit zu Beeinflussungen der Bestimmung kommt.

In besonders bevorzugten Ausführungsformen wird als Aktivator-Reagenz ein Aktivator oder ein beteiligter Komplex des extrinsischen Wegs der plasmatischen Gerinnung eingesetzt. Insbesondere können als Aktivator-Reagenzien natürlich vorkommende Aktivatoren oder Gerinnungsfaktoren wie Tissue Factor und/oder Faktor VIIa verwendet werden, wobei neben diesen natürlicherweise vorkommenden Aktivatoren oder Gerinnungsfaktoren auch andere, beispielsweise synthetisch hergestellte Aktivatoren eingesetzt werden können.

In weiteren bevorzugten Ausführungsformen wird als Aktivator-Reagenz ein Aktivator oder ein beteiligter Komplex des intrinsischen Wegs der plasmatischen Gerinnung eingesetzt. Insbesondere können als Aktivator-Reagenzien natürlich vorkommende Aktivatoren oder Gerinnungsfaktoren wie Faktor XIIa oder Substanzen, welche eine Umwandlung von Faktor X in Faktor Xa bewirken, beispielsweise Russels Viper Venom (RW-X), eingesetzt werden, wobei neben diesen natürlicherweise vorkommenden Aktivatoren auch andere, beispielsweise synthetisch hergestellte Aktivatoren eingesetzt werden können.

Das Aktivator-Reagenz wird hierbei als externes Reagenz der Probe oder den anderen Reagenzien vor oder im Verlauf der Messung zugesetzt. Weitere zum Ablauf der Faktor Xa-Aktivierung oder der Nachweisreaktion nötige Substanzen können hierbei als externes Reagenz der Probe oder den anderen Reagenzien vor oder im Verlauf der Messung zugesetzt werden oder bereits in der Blutprobe enthalten sein. So können insbesondere weitere benötigte Gerinnungsfaktoren wie Faktor V oder Antithrombin durch die zu untersuchende Blutprobe bereitgestellt werden.

Trockenchemische Reagenzien werden meist dadurch auf Testelementen aufgebracht, dass sie in Form einer Lösung strichförmig auf das Testelement aufgebracht werden und dort das Lösungsmittel entzogen wird, so dass sie dort in trockenchemischer Form vorliegen. Dies ist besonders vorteilhaft, da Reagenzien in trockenchemischer Form oft wesentlich höheren Anforderungen hinsichtlich der Haltbarkeit und Stabilität genügen als nasschemische Reagenzien. Der Transfer in die aktivere, aber auch instabilere gelöste Form erfolgt zu gegebenem Zeitpunkt durch das Wiederauflösen der trockenchemischen Reagenzien durch Flüssigkeitszufuhr, vorzugsweise durch direktes Auflösen in der zu untersuchenden Blutprobe.

Erfindungsgemäß erfolgt der Zusatz von Faktor X-Reagenz zur Probe in einem gemeinsamen Schritt mit dem Zusatz des Aktivator-Reagenz zur Probe.

In nasschemischen Reaktionen kann dies beispielsweise dadurch erfolgen, dass die beiden Reagenzien gemeinsam aus ihrem jeweiligen Lagerungskompartimenten (Vorratsbehältern) zur Probe zugegeben werden, beispielsweise mittels Pumpen oder Pipetten. Es ist aber auch möglich, dass Faktor X-Reagenz und Aktivator-Reagenz erst zusammengegeben werden und diese Reagenzmischung, in welcher Faktor Xa entsteht, dann zur Probe gegeben wird.

In einer erfindungsgemäßen Ausführungsform liegen Faktor X-Reagenz und Aktivator-Reagenz gemeinsam in einem trockenen Zustand vor und die Umwandlung von Faktor X in Faktor Xa wird erst durch den Kontakt mit der Probe bewirkt.

Durch die Verwendung von trockenchemischen Reagenzien ist es möglich, diese gemeinsam in einer inaktivierten Form zu lagern, ohne dass eine chemische Reaktion zwischen den Reagenzien stattfindet. Erst durch die Befeuchtung der trockenchemischen Reagenzien werden diese in einen Zustand gebracht, in welchem sie miteinander reagieren können. Diese "Aktivierung" der trockenchemischen Reagenzien wird in einer bevorzugten Ausführungsform durch die flüssige Blutprobe selbst bewirkt.

In einer besonders bevorzugten Ausführungsform liegen Faktor X-Reagenz, Aktivator-Reagenz und Nachweisreagenz gemeinsam als trockenchemische Reagenzien vor und die Umwandlung von Faktor X in Faktor Xa wird erst durch den Kontakt mit der Probe bewirkt.

Da es durch die Verwendung trockenchemischer Reagenzien möglich ist, diese gemeinsam in einer inaktivierten Form zu lagern, ohne dass eine chemische Reaktion zwischen den Reagenzien stattfindet, können mit diesen erfindungsgemäßen Verfahren und Vorrichtungen alle zur Bestimmung der FaktorXa-Inhibitoren notwendigen Reagenzien in einem gemeinsamen Kompartiment gelagert werden, ohne dass es zu einem Faktor Xa-bedingten vorzeitigen Thrombinsubstratabbau kommt. Erst durch die Zugabe der flüssigen Probe kommt es zu Umwandlung von Faktor X in Faktor Xa mit weitgehend definiertem Startpunkt und zeitlichem Verlauf, so dass schließlich eine bekannte Menge Faktor Xa in der Probe vorliegt, welche die Grundlage der Bestimmung von FaktorXa-Inhibitoren nach dem erfindungsgemäßen Prinzip bildet. Hierdurch lassen sich prinzipiell mit dem Fachmann bekannten Methoden alle zur Bestimmung

von FaktorXa-Inhibitoren notwendigen Reagenzien in einem einzigen trockenchemischen Reagenzstrich oder -spot vereinen, was eine einfache und kostengünstige Produktion und eine einfache und weniger fehleranfällige Handhabung ermöglichen kann.

Die erfindungsgemäßen Verfahren und Vorrichtungen zur Bestimmung von Faktor Xa-Inhibitoren können insbesondere vorteilhaft zur Bestimmung von Heparinen, insbesondere fraktionierten oder niedermolekularen Heparinen, eingesetzt werden. Weitere Faktor Xa-Inhibitoren, welche mit den erfindungsgemäßen Verfahren und Vorrichtungen vorteilhaft bestimmt werden können, können insbesondere indirekte selektive oder direkte Faktor Xa-Inhibitoren sein.

Als Faktor Xa-Inhibitoren im Sinne der Erfindung können alle Substanzen angesehen werden, welche direkt oder indirekt die Aktivität des Faktors Xa beeinflussen, insbesondere erniedrigen und somit den Ablauf der Blutgerinnungskaskade beeinflussen, insbesondere verzögern. Direkte Faktor Xa-Inhibitoren wirken direkt auf den Faktor Xa und beeinflussen so dessen Aktivität, während indirekte Faktor Xa-Inhibitoren nicht direkt mit dem Faktor Xa wechselwirken, sondern dessen Menge und Bildung beeinflussen bzw. für ihre Aktivierung einen Cofaktor benötigen.. Dies können beispielsweise Antikoagulantien sein, welche auf Gerinnungsfaktoren wirken, welche in der Gerinnungskaskade vor Faktor Xa angesiedelt sind und zu dessen Bildung und/oder Regulierung beitragen. Beispiele für indirekte selektive Faktor Xa-Inhibitoren sind bestimmte Pentasaccharide wie Fondaparinux und Idraparinux, welche im Zusammenspiel mit Antithrombin III wirken und anders als die Heparine selektiv für Faktor Xa sind. Beispiele für direkte Faktor Xa-Inhibitoren ist Otamixaban oder Razaxaban.

Ein weiterer Aspekt der vorliegenden Erfindung betrifft Testelemente mit einem elektrochemischen Sensor auf trockenchemischer Basis zur Bestimmung von Faktor Xa-Inhibitoren, insbesondere unfraktionierten Heparinen, fraktionierten oder niedermolekularen Heparinen, indirekten selektiven Faktor Xa-Inhibitoren oder direkten Faktor Xa-Inhibitoren, der auf einem inerten Träger mindestens zwei Elektroden aufweist und ein Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, sowie zumindest eine bestimmte Menge von Faktor X-Reagenz und ein Aktivator-Reagenz enthält.

Solche erfindungsgemäßen Testelemente eignen sich besonders, die zuvor beschriebenen erfindungsgemäßen Verfahren zur Bestimmung von Faktor Xa-Inhibitoren in Blutproben durchzuführen.

Erfindungsgemäße Testelemente sind elektrochemische Testelemente auf trockenchemischer Basis. Solche Testelemente enthalten mindestens zwei Elektroden, von denen mindestens eine Elektrode eine so genannte Arbeitselektrode ist. Sie enthalten nicht notwendigerweise eine klassische Referenzelektrode, wie beispielsweise eine Ag/AgCl-Referenzelektrode, sondern lediglich mindestens eine Arbeits- und eine Gegenelektrode. Die Elektroden können aus allen gängigen Elektrodenmaterialien, beispielsweise Metallen, Edelmetallen, Legierungen oder Graphit aufgebaut sein, vorzugsweise aus Edelmetallen, wie Gold oder Palladium, oder Graphit. Die unterschiedlichen Elektroden des Testelements können aus gleichen oder unterschiedlichen Materialien bestehen. In einer besonders bevorzugten Ausführungsform enthält das Testelement eine Arbeitselektrode und eine Gegenelektrode, die beide aus Gold bestehen.

Konkrete Ausgestaltungen solcher elektrochemischer Testelemente, insbesondere hinsichtlich Wahl der Materialien, Anordnung der Elektroden und Reagenzien sowie Herstellung der Testelemente, sind im Stand der Technik, beispielsweise in US 5762770, US 6270637 oder WO 01/63271 beschrieben und dem Fachmann bekannt.

Die Reagenzien werden in bevorzugten Ausführungsformen in Form eines oder mehrerer Reagenzstriche auf die Elektroden aufgebracht. Diese können sich teilweise oder komplett überdecken. Weiterhin ist es erfindungsgemäß möglich, die Reagenzien nicht direkt auf die Elektroden, sondern in der Nähe der Elektroden, beispielsweise neben den Elektroden auf einem flachen Substrat, aufzubringen und zu trocknen. In diesem Fall werden die Reagenzien mit der Probe während der Messung zu den Elektroden transportiert. Alternativ dazu ist es möglich, die Reagenzien in porösen Materialien wie zum Beispiel Vliesen, Papieren, Membranen und dergleichen unterzubringen, beispielsweise ablösbar zu imprägnieren. Diese Materialien müssen dabei von der Probe vor dem Kontakt mit den Elektroden durchströmt werden und dabei die Reagenzien in die Probe abgeben können. Es ist auch möglich, einzelne Bestandteile der Reagenzien auf unterschiedliche Kompartimente des Testelements aufzubringen, beispielsweise das Nachweisreagenz auf oder direkt neben die Elektrode, ein Faktor X-Reagenz gemeinsam mit einem Aktivator-Reagenz aber räumlich getrennt davon (z.B. weiter entfernt von der Elektrode), oder in unterschiedliche Schichten (z. B. Membranen oder Vliese) zu imprägnieren.

Erfindungsgemäß enthält das Testelement Faktor X-Reagenz und Aktivator-Reagenz in trockenchemischer Form, welches die Umwandlung von Faktor X in Faktor Xa hervorruft, wobei das Aktivator-Reagenz auf dem Testelement gemeinsam mit dem Faktor X-Reagenz, optional auch gemeinsam mit dem Nachweisreagenz, vorliegt und wobei die Umwandlung von Faktor X in Faktor Xa erst durch den Kontakt mit der Probe bewirkt wird. Dadurch, dass Faktor X-Reagenz und Aktivator-Reagenz in trockenchemischer Form auf dem Testelement vorliegen, liegen diese in einer Form vor, in welcher sie nicht miteinander reagieren können. Dadurch kann Faktor X-Reagenz und Aktivator-Reagenz gemeinsam in einem Kompartiment gelagert werden, ohne dass Faktor Xa gebildet wird. Erst durch den Zusatz von Flüssigkeit, insbesondere durch den Kontakt mit der Probenflüssigkeit, können die beiden Reagenzien miteinander reagieren und es kann zur Umwandlung von Faktor X in Faktor Xa kommen.

In einer bevorzugten Ausführungsform wird dies beispielsweise dadurch erreicht, dass Faktor X-Reagenz und Aktivator-Reagenz in Form eines gemeinsamen Reagenzstriches zusammen auf dem Testelement angeordnet sind. Das Nachweisreagenz kann bei dieser Ausführungsform in einem weiteren Kompartiment vorliegen, insbesondere einem weiteren Reagenzstrich, welcher allerdings mit dem Reagenzstrich des kombinierten Aktivator- /Faktor X-Reagenzes zumindest teilweise in Kontakt stehen kann.

In einer besonders bevorzugten Ausführungsform kann auch das Nachweisreagenz dem Faktor X-Reagenz und dem Aktivator-Reagenz zugesetzt sein, so dass alle zur Bestimmung von Faktor Xa-Inhibitoren benötigten Reagenzien in einem einzigen Kompartiment, insbesondere einem einzigen Reagenzstrich, auf dem Testelement vorliegen können. Wie zuvor ausgeführt, ist es durch die Verwendung trockenchemischer Reagenzien möglich, Faktor X-Reagenz und Aktivator-Reagenz gemeinsam in einem Kompartiment zusammenzubringen, ohne dass Faktor Xa gebildet wird. Hierdurch ist es nun möglich, auch das Nachweisreagenz zu dieser Reagenzienkombination zu geben, da ohne aktivierten Faktor Xa auch kein vorzeitiger Faktor Xa-bedingter Abbau des Nachweisreagenzes erfolgen kann. Insbesondere ist es durch diese erfindungsgemäße Lösung möglich, alle zur Bestimmung von Faktor Xa-Inhibitoren notwendigen Reagenzien in einem einzigen Kompartiment aufzubringen, wodurch einfachere und kostengünstiger herzustellende Testelementaufbauten möglich sind.

Ein weiterer Aspekt der vorliegenden Erfindung betrifft elektrochemische Testelement-Analysesysteme, welche zumindest ein Testelement gemäß den Patentansprüchen und ein Strom- oder Spannungsmessgerät enthalten. Durch die Verwendung solcher Messgeräte können die beim Ablauf der Faktor Xa-Inhibitor-Bestimmung entstehenden elektrogenen Thrombinsubstrat-Spaltprodukte, insbesondere Phenylendiamin, mittels des an den Elektroden stattfindenden Stromflusses erfasst werden. In besonders bevorzugten Ausführungsformen wird der Verlauf des Stromflusses über die Zeit erfasst. Die elektrochemische Bestimmung erfolgt vorzugsweise quasi-potentiostatisch, bevorzugt mit einem 2-Elektrodensystem, bei dem die eine Elektrode gleichzeitig als Referenz- und Gegenelektrode, die andere Elektrode als Arbeitselektrode geschaltet ist. An dieses 2-Elektrodensystem wird eine konstante Spannung angelegt und der Strom über die Zeit gemessen. Dieses Verfahren wird auch als amperometrisches Messverfahren bezeichnet. Hierbei wird der zeitliche Stromverlauf erfasst und ermittelt, nach welcher Zeitspanne ab dem Start der Bestimmungsreaktion der gemessene Strom einen vorbestimmten Schwellenwert überschreitet. Diese Zeitspanne ist ein Maß für die Faktor Xa-bedingte Thrombinsubstratumsetzung im Laufe der Gerinnungsreaktion, welche wiederum von der Menge des in der Probe vorhandenen Faktor Xa-Inhibitors abhängt.

Neben dem beschriebenen amperometrischen Messverfahren können auch voltametrische Messverfahren eingesetzt werden. Hierbei wird keine konstante Spannung zwischen den Elektroden geregelt, sondern die Spannung wird linear von einem Startwert zu einem Endwert verändert und anschließend wieder zum Startwert zurück gefahren. Dieser Vorgang kann mehrfach über die gesamte Messdauer wiederholt werden. Beim voltametrischen Verfahren wird der Strom gegen die Spannung aufgetragen und man erhält dann entsprechend den Wiederholungen ineinander geschachtelte Strom-Spannungskurven (Zyklovoltamogramme). Bei geeignetem Spannungsbereich bilden sich Oxidationspeaks und Reduktionspeaks des Elektronenüberträgers in diesen Kurven ab. Die Höhe dieser Peaks ist direkt proportional der Konzentration des Elektronenüberträgers, sofern nicht weitere redoxaktive Substanzen im überdeckten Potentialbereich mit oxidiert oder reduziert werden und damit einen zusätzlichen Strombeitrag liefern. Bei der Messung einer Konzentrationsänderung kann eine solche Störung gegebenenfalls vernachlässigt werden. Trägt man nun die Stromwerte der Peak-Maxima oder die durch die Kurven eingeschlossenen Flächen (die dem Ladungsumsatz entsprechen) der einzelnen Strom-Spannungsschleifen über die Zeit auf, erhält man ebenfalls ein Bild der Konzentrationsänderung des Elektronenüberträgers über die Messdauer in einem durch die Zyklusdauer gegebenen Zeitraster. Diese Information kann dann - wie bei den amperometrischen Verfahren - zur Bestimmung des Faktor Xa-Inhibitors herangezogen werden. Solche Verfahren sind beispielsweise in WO 01/63271 beschrieben.

Die erfindungsgemäßen Verfahren und Vorrichtungen können insbesondere zur Bestimmung von Faktor Xa-Inhibitoren in Blutproben eingesetzt werden. Im Sinne der vorliegenden Anmeldung umfasst der Begriff Blutprobe nicht nur unbehandeltes Blut (Vollblut), sondern auch Blutderivate oder Blutprodukte, welche durch physikalische und/oder chemische und/oder biochemische Weiterbehandlung aus nativen Blutproben gewonnen werden können. Als Blutderivate können im Sinne der vorliegenden Anmeldung insbesondere Serum oder Plasma angesehen werden. Unter Bestimmung von Faktor Xa-Inhibitoren kann im Sinne der vorliegenden Anmeldung jede qualitative, quantitative oder semiquantitative Bestimmung von Faktor Xa-Inhibitoren gelten.

### Ausführungsbeispiele

Die Erfindung wird durch nachfolgende exemplarische Beispiele und Abbildungen näher erläutert. Beispiele die Faktor X-und Aktivator-Reagenz nicht in Kombination enthalten, dienen Vergleichszwecken.
Figur 1 zeigt exemplarisch das primäre Messergebnis einer elektrochemischen Faktor Xa-Inhibitor-Bestimmung entsprechend den erfindungsgemäßen Verfahren.
Figur 2 zeigt die Ergebnisse elektrochemischer Thrombinbestimmungen bei unterschiedlichen Anordnungen der Reagenzstriche eines Aktivator-Reagenzes und einer Nachweisreagenz/Faktor X-Reagenz-Kombination auf einem Testelement

### Beispiel 1: Exemplarischer Aufbau eines erfindungsgemäßen Testelements

Ein möglicher Aufbau eines erfindungsgemäßen Testelements und dessen Fertigung wird im Folgenden exemplarisch beschrieben.

Das inerte Trägermaterial kann beispielsweise aus einer Polyesterfolie, beispielsweise einer Melinexfolie, bestehen. Auf diese können mit verschiedensten Verfahren Elektrodenstrukturen aufgebracht werden, beispielsweise durch Bedampfung der Trägerfolie mit Gold und anschließender Laserablation der gewünschten Elektrodenstrukturen, wobei dem Fachmann auch noch andere Verfahren wie beispielsweise Ätzverfahren oder Druckverfahren zur Herstellung dieser Elektrodenstrukturen geläufig sind. Optional können anschließend bestimmte Teile der Elektrodenstrukturen mit isolierenden Schichten versehen werden.

Auf das Trägermaterial mit seinen Elektrodenstrukturen werden nun die zur Bestimmung benötigten Reagenzien aufgebracht. Dies kann mit verschiedenen dem Fachmann bekannten Methoden geschehen, insbesondere mit Druck- oder Rakelverfahren, bei welchen die Reagenzien in gelöster Form in Form von Reagenzstrichen auf das Testelement aufgebracht werden und anschließend das Lösungsmittel zumindest teilweise entzogen wird, so dass die Reagenzien in trockenchemischer, d.h. inaktivierter Form, auf dem Testelement vorliegen. Solche Verfahren sind beispielsweise in WO 01/63271 beschrieben.

Üblicherweise werden bei solchen Beschichtungsverfahren die Reagenzien in einem Grundansatz gelöst, welcher Substanzen enthält, welche eine möglichst optimale Verarbeitung der Reagenzlösung ermöglichen.

Eine exemplarische Zusammensetzung eines solchen Grundansatzes ist beispielsweise (gelöst in Aqua bidest):

| Konzentration (in Klammern: erfindungsgemäß möglicher Bereich) | Substanz | Funktion der Substanz | Lieferant |
|---|---|---|---|
| 3,5 g/l (0-50 g/l) | Keltrol F | Filmbildner | Roche Diagnostics GmbH |
| 5 g/l (0-75 g/l) | Hydroxypropyl-zellulose | Verdicker | Hercules |
| 1 g/l (0-10 g/l) | Mega 8 | Detergenz | Roche Diagnostics GmbH |
| 20 g/l (0-200 g/l) | Mowiol | Filmbildner | Kuraray |
| | Grundpuffer | | |
| 120 mM (10 - 500 mM) | HEPES | pH-Puffer | Sigma |
| 10 g/l (0,1-100 g/l) | RPLA 4 (Serumalbumin) | Schutzprotein | Roche Diagnostics GmbH |
| 30 g/l (0-200 g/l) | Glycin | Stabilisator, Lösungsvermittler | Roche Diagnostics GmbH |
| 75 g/l (0-300 g/l) | Sucrose | Stabilisator, Lösungsvermittler | Roche Diagnostics GmbH |

Zu diesem Grundansatz werden nun jeweils die spezifischen Reagenzkomponenten (z.B. Thrombinsubstrat, Faktor X, Aktivator-Reagenz; einzeln oder auch in Kombination) zugemischt und räumlich definiert, beispielsweise als Reagenzstriche oder -spots, auf das Testelement aufgebracht. Nach Entzug des Lösungsmittels liegen nun definierte und stabile Reagenzstriche oder -spots auf dem Testelement vor, in welchen die Reagenzien in stabiler Form über einen langen Zeitraum ohne Aktivitätsverlust gelagert werden können. In bevorzugten Ausführungsformen werden auf die Testträger weiterhin seitliche Abstandshalter und eine Deckelfolie aufgebracht, welche einen Kapillarkanal definieren, durch welcher ein weitgehend definiertes Volumen der Probe aufgenommen und zu den Reagenzkompartimenten und Elektroden transportiert werden kann.

### Beispiel 2: Exemplarische Durchführung einer elektrochemischen Faktor Xa-Inhibitor-Bestimmung_entsprechend den erfindungsgemäßen Verfahren und Vorrichtungen

Auf ein erfindungsgemäßes Testelement, wie es vorzugsweise nach Beispiel 1 hergestellt werden kann, wird zur Durchführung der Faktor Xa-Inhibitor-Bestimmung eine ausreichende Menge, beispielsweise 1 - 1000 µl, bevorzugt ca. 10 µl, des zu untersuchenden Blutes in einer Weise aufgebracht, dass es mit den auf dem Testelement befindlichen Reagenzkompartimenten und Elektroden in Kontakt kommt. Dies kann bevorzugterweise durch das Vorhandensein eines Kapillarkanals auf dem Testelement und geeignete Positionierung der Reagenzkompartimente und Elektroden auf dem Testelement innerhalb dieses Kapillarkanals bewirkt werden. In bevorzugten Ausführungsformen können Probenflüssigkeit, Testelement und/oder sonstige zur Faktor Xa-Inhibitor-Bestimmung notwendigen Vorrichtungen auf eine bestimmte Temperatur, insbesondere eine Temperatur von ca. 37°C, temperiert sein. Das Testelement wird zur Erfassung der elektrochemischen Nachweisreaktionen an ein geeignetes Messgerät angeschlossen, beispielsweise ein Strommesssystem, welches insbesondere den zeitlichen Verlauf des Stromflusses erfassen und speichern kann.

Figur 1 zeigt exemplarisch den zeitlichen Verlauf einer elektrochemischen Faktor Xa-Inhibitor-Bestimmung entsprechend den erfindungsgemäßen Verfahren. Hierbei wurde ein Testelement entsprechend Beispiel 1 eingesetzt.

Die Kurve zeigt den zeitlichen Verlauf der gemessenen Stromstärke, bedingt durch die Oxidation des Phenylendiamins nach dessen Thrombin-induzierter Abspaltung aus dem Thrombin-Substrat. Auf der x-Achse ist die Zeit t in Sekunden nach der Auftragung der Blutprobe, auf der y-Achse die zum jeweiligen Zeitpunkt bestimmte Stromstärke I in Nanoampere aufgetragen. Die Kurve durchläuft zunächst ein Minimum, steigt dann mit zunehmender Oxidation des Phenylendiamins an, erreicht schließlich ein Maximum und fällt dann aufgrund der Substratverarmung kontinuierlich ab. Die Auswertung solcher zeitlichen Stromverläufe zur Bestimmung von zeitlichen Messgrößen, welche den Ablauf der Nachweisreaktion widerspiegeln, erfolgt beispielsweise mittels eines Schwellwertalgorithmus. Dieser Algorithmus addiert auf das errechnete Minimum einen Schwellwert, beispielsweise 60 nA, der für alle Messungen beibehalten wird. Als zeitliche Messgröße für die Thrombinumsetzung kann die Zeit nach Erreichen des Minimums bestimmt werden, bis zu welcher dieser Schwellwert erreicht wird. Da die so ermittelten zeitlichen Messgrößen für die Thrombinumsetzung von der Aktivität/Konzentration des aktivierten Faktors Xa und diese wiederum von der Menge bzw. Konzentration evtl. Faktor Xa-Inhibitoren abhängig sind, kann von den so ermittelten Zeitwerten auf das Vorhandensein, das Über- oder Unterschreiten einer bestimmten Konzentration bzw. die Menge oder die Konzentration eines Faktor Xa-Inhibitors geschlossen werden. Hierzu können beispielsweise Kurven mit Proben bekannter Faktor Xa-Inhibitor-Konzentration erstellt werden, welche diese Konzentrationen mit deren erfindungsgemäß ermittelten Zeitparametern in Korrelation setzen.

Weiterhin können aus diesen bestimmten zeitlichen Messgrößen weitere damit und mit der Menge eines evtl. vorhandenen Faktor Xa-Inhibitors korrelierende Messgrößen, insbesondere Gerinnungszeiten, unter Verwendung geeigneter Algorithmen ermittelt werden.

### Beispiel 3: Einfluss der Anordnungen der Reagenzstriche auf dem Testelement, welches Faktor X-Reagenz und ein Aktivator-Reagenz enthält

Figur 2 zeigt die Ergebnisse elektrochemischer Thrombinbestimmungen bei unterschiedlichen Anordnungen der Reagenzstriche eines Aktivator-Reagenzes und einer Nachweisreagenz/Faktor X-Reagenz-Kombination auf einem Testelement. Dargestellt ist jeweils der zeitliche Verlauf der gemessenen Stromstärke, wobei auf der x-Achse die Zeit t in Sekunden nach der Auftragung der Blutprobe und auf der y-Achse die zum jeweiligen Zeitpunkt bestimmte Stromstärke I in Nanoampere aufgetragen ist. Im vorliegenden Beispiel enthielt der Reagenzstrich der Nachweisreagenz/Faktor X-Reagenz-Kombination 0,8ml reduziertes Chromozyms TH und 38,1µl Faktor X (factor X der Firma american diagnostica; entsprechend 2U/mI Faktor Xa), der Reagenzstrich des Aktivator-Reagenz 80µl Russels Viper Venom (Russels Viper Venom (RVV-X) der Firma Pentapharm Ltd.) und 0,04% Phospholipide (PHL).

Die Reagenzstriche wurden in folgenden Varianten aufgetragen:
Kurve A: Zuerst wurde der Reagenzstrich der Nachweisreagenz/Faktor X-Reagenz-Kombination aufgetragen, über diesen dann der Reagenzstrich des Aktivator-Reagenzes.
Kurve B: Zuerst wurde der Reagenzstrich der Nachweisreagenz/Faktor X-Reagenz-Kombination aufgetragen, über diesen dann der Reagenzstrich des Aktivator-Reagenzes, wobei die Auftragung des Reagenzstriches des Aktivator-Reagenzes versetzt erfolgte, so dass dieser zumindest teilweise in Flussrichtung der Probe vor dem Reagenzstrich der Nachweisreagenz/Faktor X-Reagenz-Kombination liegt und diesen zumindest teilweise überdeckt.

Beide Kurven in Figur 2 zeigt keinen Versatz des Minimums zu höheren Stromwerten sowie einen niedrigen Eingangsstrom. Dies deutet darauf hin, dass mit diesen bevorzugten erfindungsgemäßen Verfahren und Vorrichtungen der vorzeitige Substratabbau durch die zumindest teilweise räumliche Trennung der Reagenzien in zwei Kompartimente, insbesondere Reagenzstriche, größtenteils unterbunden werden kann.

Ein in Flussrichtung der Probe vor dem Reagenzstrich der Nachweisreagenz/Faktor X-Reagenz-Kombination versetzter Auftrag des Reagenzstrichs des Aktivator-Reagenz (Kurve B) zeigt gegenüber dem Auftrag des Reagenzstrichs des Aktivator-Reagenzes direkt über dem Reagenzstrich der Nachweisreagenz/Faktor X-Reagenz-Kombination (Kurve A) geringere Strommaxima sowie verlangsamte Reaktionskinetiken. Ein Auftrag des Reagenzstrichs des Aktivator-Reagenz direkt über dem Reagenzstrich der Nachweisreagenz/Faktor X-Reagenz-Kombination sind vor allem aufgrund der rascheren Reaktionskinetiken für die erfindungsgemäße Bestimmung von Faktor Xa-Inhibitoren, insbesondere bei diagnostischen Anwendungen, besonders vorteilhaft.

## Patentansprüche

1. Verfahren zur Bestimmung von Faktor Xa-Inhibitoren in Blutproben,
**dadurch gekennzeichnet, dass**
a) eine Probe des zu untersuchenden Blutes mit einem Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, enthält, und mit einem Reagenz enthaltend eine bekannte Menge von Faktor X und einem Aktivator-Reagenz, welches die Umwandlung von Faktor X in Faktor Xa hervorruft, in Kontakt gebracht wird, wobei der Zusatz des Reagenz enthaltend eine bekannte Menge von Faktor X zur Probe in einem gemeinsamen Schritt mit dem Zusatz des Aktivator-Reagenz zur Probe erfolgt,
b) die Menge oder Aktivität der elektrogenen Substanz, welche durch die Faktor Xavermittelte Thrombinaktivierung vom Thrombinsubtrat abgespalten wird,
und/oder deren zeitlicher Verlauf als Messsignal mit elektrochemischen Methoden bestimmt wird, und
c) anhand dieses Messsignals die Menge des Faktor Xa-Inhibitors in der Probe des zu untersuchenden Blutes oder eine damit korrelierende Messgröße, insbesondere eine damit korrelierende Gerinnungszeit, ermittelt wird.

2. Verfahren gemäß Anspruch 1,
**dadurch gekennzeichnet, dass**
als Aktivator-Reagenz ein Aktivator oder ein beteiligter Komplex des extrinsischen Wegs der plasmatischen Gerinnung, insbesondere Tissue Factor und/oder Faktor VIIa, oder ein Aktivator oder ein beteiligter Komplex des intrinsischen Wegs der plasmatischen Gerinnung, insbesondere Faktor XIIa,oder eine Substanz, welche eine Umwandlung von Faktor X in Faktor Xa hervorruft, eingesetzt wird.

3. Verfahren gemäß Anspruch 1,
**dadurch gekennzeichnet, dass**
Reagenz enthaltend eine bekannte Menge von Faktor X und Aktivator-Reagenz gemeinsam in einem trockenen Zustand vorliegen und die Umwandlung von Faktor X in Faktor Xa erst durch den Kontakt mit der Probe bewirkt wird.

4. Verfahren gemäß Anspruch 3,
**dadurch gekennzeichnet, dass**
Reagenz enthaltend eine bekannte Menge von Faktor X, Aktivator-Reagenz und Nachweisreagenz gemeinsam in einem trockenen Zustand vorliegen und die Umwandlung von Faktor X in Faktor Xa erst durch den Kontakt mit der Probe bewirkt wird.

5. Verfahren gemäß einem der vorherigen Ansprüche,
**dadurch gekennzeichnet, dass**
der Faktor Xa-Inhibitor ein Heparin, insbesondere ein fraktioniertes oder niedermolekulares Heparin, ein indirekter selektiver oder ein direkter Faktor Xa-Inhibitor ist.

6. Testelement mit einem elektrochemischen Sensor auf trockenchemischer Basis zur Bestimmung von Faktor Xa-Inhibitoren, insbesondere unfraktionierten Heparinen und fraktionierten oder niedermolekularen Heparinen, indirekten selektiven Faktor Xa-Inhibitoren und direkten Faktor Xa-Inhibitoren, der auf einem inerten Träger mindestens zwei Elektroden aufweist und ein Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, sowie zumindest ein Reagenz enthaltend eine bekannte Menge von Faktor X und ein Aktivator-Reagenz, welches die Umwandlung von Faktor X in Faktor Xa hervorruft, als trockenchemische Reagenzien enthält, wobei das Aktivator-Reagenz auf dem Testelement gemeinsam mit dem Reagenz enthaltend eine bekannte Menge von Faktor X, optional auch gemeinsam mit dem Nachweisreagenz, vorliegt, so dass die Umwandlung von Faktor X in Faktor Xa erst durch den Kontakt mit der Probe bewirkt wird.

7. Elektrochemisches Testelement-Analysesystem,
enthaltend zumindest ein Strom- oder Spannungsmessgerät und ein Testelement gemäß Anspruch 6.

## Claims

1. Method for determining factor Xa inhibitors in blood samples **characterized in that**
a) a sample of the blood to be analysed is brought into contact with a detection reagent which contains at least one thrombin substrate which consists of a peptide residue that can be cleaved by thrombin and which is amidically bound via the carboxyl end to an electrogenic substance, and with a reagent containing a known amount of factor X and an activator reagent which induces the conversion of factor X into factor Xa, where the addition of the reagent containing a known amount of factor X to the sample is carried out in a common step with the addition of the activator reagent to the sample,
b) the amount or activity of the electrogenic substance which is cleaved from the thrombin substrate by the factor Xa-mediated thrombin activation and/or its time course is determined as the measurement signal using electrochemical methods, and
c) the amount of the factor Xa inhibitor in the sample of the blood to be analysed or a measured quantity that correlates therewith in particular a clotting time that correlates therewith is determined on the basis of this measurement signal.

2. Method according to claim 1,
**characterized in that**
an activator or a complex involved in the extrinsic pathway of plasmatic coagulation, in particular tissue factor and/or factor VIIa, or an activator or a complex involved in the intrinsic pathway of plasmatic coagulation in particular factor XIIa or a substance which induces a conversion of factor X into factor Xa is used as the activator reagent.

3. Method according to claim 1,
**characterized in that**
the X reagent containing a known amount of factor Xa and activator reagent are present together in a dry state and factor X is not converted into factor Xa until contact with the sample.

4. Method according to claim 3,
**characterized in that**
the reagent containing a known amount of factor Xa, activator reagent and detection reagent are present together in a dry state and factor X is not converted into factor Xa until contact with the sample.

5. Method according to one of the previous claims
**characterized in that**
the factor Xa inhibitor is a heparin, in particular a fractionated or low-molecular-weight heparin, an indirect selective or a direct factor Xa inhibitor.

6. Test element with an electrochemical sensor on a dry chemistry basis for determining factor Xa inhibitors, in particular unfractionated heparins, fractionated or low-molecular-weight heparins, indirect selective factor Xa inhibitors and direct factor Xa inhibitors which, on an inert carrier, has at least two electrodes and a detection reagent which contains at least one thrombin substrate which consists of a peptide residue that can be cleaved by thrombin and is bound amidically via the carboxyl end to an electrogenic substance, as well as at least a reagent containing a known amount of factor X and an activator reagent which induces the conversion of factor X into factor Xa as dry chemistry reagents, wherein the activator reagent is present on the test element together with the reagent containing a known amount of factor Xa and optionally also together with the detection reagent so that the conversion of factor X into factor Xa does not take place until contact with the sample.

7. Electrochemical test element analytical system,
containing
at least one device for measuring current or voltage and a test element according to claim 6.

## Revendications

1. Procédé pour la détermination d'inhibiteurs du facteur Xa dans des échantillons de sang, **caractérisé en ce que**
a) un échantillon du sang à analyser est mis en contact avec un réactif de détection, qui contient au moins un substrat de thrombine, qui est constitué d'un reste peptidique, qui peut être dissocié par la thrombine et qui est lié amidiquement, via l'extrémité carboxyle, à une substance électrogène et avec un réactif contenant une quantité connue de facteur X et un réactif activateur, qui provoque la transformation du facteur X en facteur Xa, l'addition du réactif contenant une quantité connue du facteur X à l'échantillon ayant lieu lors d'une étape commune avec l'addition du réactif activateur à l'échantillon,
b) la quantité ou l'activité de la substance électrogène, qui est dissociée du substrat de thrombine par l'activation de la thrombine induite par le facteur Xa, et/ou leur allure dans le temps sont déterminées comme signal de mesure par des procédés électrochimiques, et
c) à l'aide de ce signal de mesure, la quantité d'inhibiteur du facteur Xa dans l'échantillon du sang à analyser ou une grandeur de mesure qui corrèle avec celle-ci, en particulier un temps de coagulation corrélant avec celle-ci, est déterminée.

2. Procédé selon la revendication 1, **caractérisé en ce qu'**on utilise, comme réactif activateur, un activateur ou un complexe associé de la voie extrinsèque de la coagulation plasmatique, en particulier le facteur tissulaire et/ou le facteur VIIa, ou un activateur ou un complexe associé de la voie intrinsèque de la coagulation plasmatique, en particulier le facteur Xlla ou une substance, qui provoque une transformation du facteur X en facteur Xa.

3. Procédé selon la revendication 1, **caractérisé en ce que** le réactif, contenant une quantité connue de facteur X, et le réactif activateur se trouvent ensemble à l'état sec et la transformation du facteur X en facteur Xa ne se produit que par le contact avec l'échantillon.

4. Procédé selon la revendication 3, **caractérisé en ce que** le réactif, contenant une quantité connue de facteur X, le réactif activateur et le réactif de détection se trouvent ensemble à l'état sec et la transformation du facteur X en facteur Xa ne se produit que par le contact avec l'échantillon.

5. Procédé selon l'une quelconque des revendications précédentes, **caractérisé en ce que** l'inhibiteur du facteur Xa est une héparine, en particulier une héparine fractionnée ou de bas poids moléculaire, un inhibiteur indirect sélectif ou direct du facteur Xa.

6. Elément test présentant un capteur électrochimique à base de chimie sèche pour la détection d'inhibiteurs du facteur Xa, en particulier d'héparines non fractionnées et d'héparines fractionnées ou de bas poids moléculaire, d'inhibiteurs indirects sélectifs du facteur Xa et d'inhibiteurs directs du facteur Xa, qui présente, sur un support inerte, au moins deux électrodes et qui contient, comme réactifs de chimie sèche, un réactif de détection, qui contient au moins un substrat de thrombine, constitué par un reste peptidique, qui peut être dissocié par la thrombine et qui est lié amidiquement via l'extrémité carboxyle à une substance électrogène, ainsi qu'au moins un réactif contenant une quantité connue de facteur X et un réactif activateur, qui provoque la transformation du facteur X en facteur Xa, le réactif activateur se trouvant sur l'élément test ensemble avec le réactif contenant une quantité connue de facteur X, éventuellement aussi ensemble avec le réactif de détection, de telle sorte que la transformation du facteur X en facteur Xa ne se produit que par le contact avec l'échantillon.

7. Système d'analyse électrochimique à élément test, contenant au moins un appareil de mesure du courant ou de la tension et un élément test selon la revendication 6.
